Europäisches Patentamt

(19) European Patent Office

Office européen des brevets

(11) Publication number: **0 446 645 A1**

# (12) EUROPEAN PATENT APPLICATION

(21) Application number: **91102166.5**

(22) Date of filing: **15.02.91**

(51) Int. Cl.⁵: **A61B 8/12**

(30) Priority: **20.02.90 US 482267**

(43) Date of publication of application:
**18.09.91 Bulletin 91/38**

(84) Designated Contracting States:
**DE FR GB IT**

(71) Applicant: **ACOUSTIC IMAGING TECHNOLOGIES CORPORATION**
**10027 South 51st Street**
**Phoenix, Arizona 85044 (US)**

(72) Inventor: **Kopel, LeRoy A.**
**5304 South Marine Drive**
**Tempe, Arizona 85283 (US)**

(74) Representative: **Kasseckert, Rainer**
**DORNIER GMBH Kleeweg 3**
**W-7990 Friedrichshafen 1 (DE)**

(54) Method and apparatus for ultrasonically probing a prostate.

(57)    An ultrasonic probe apparatus configured to a patient's pelic anatomy and method of using same is disclosed. The apparatus includes a housing (20) which has a straight portion (18) and curvilinear end portion (22) which has an outer surface (24) convexly configured, such as to describe an arc of a circle. Ultrasonic transducer means (28) are arrayed along the outer surface. Ultrasonic transmissions directed toward the prostate, or other area to be probed, along a plurality of transmission axes enable the apparatus to provide a field of view (B-B) of substantially all of the desired area. For prostate examination, the probe apparatus is oriented to face the prostate (P) after insertion into the pelvic anatomy and the ultrasonic transmissions are directed toward the prostate to provide the desired field of view (B-B) and image of the entire prostate.

EP 0 446 645 A1

FIG. 2.

# METHOD AND APPARATUS FOR ULTRASONICALLY PROBING A PROSTATE

## Reference to Co-pending Application

Reference is made to co-pending U.S. Application S.N. 07/443,752, filed November 30, 1989, in the name of Bradley R. Herres, entitled "Ultrasonic Imaging Method and Apparatus", assigned to the same assignee as the present application. The disclosure of said application S.N. 07/443,752, is hereby incorporated herein by reference.

## Field of the Invention

The present invention relates to a method and apparatus for ultrasonically probing a patient's internal cavities. More particularly, the invention relates to providing and utilizing an ultrasonic probe apparatus which conforms to a patient's pelvic anatomy and which is capable of imaging the entire prostate. The invention is also useful as a type of vaginal probe.

## Background of the Invention

Ultrasonic diagnostics have been used to obtain acoustic images of body tissue. To perform such acoustic imaging, ultrasonic waves or beams are generated, such as by suitable ultrasonic transducer means, which are directed to the particular body tissue to be examined. The "echoes" received by the ultrasonic transducer means are processed in an image format suitable for display. It is desirable for the ultrasonic diagnostic device to produce a sufficiently large field of view to encompass the entire body tissue to be scanned and viewed. The scan image is obtained by repeatedly transmitting and receiving the ultrasonic energy. Generally, for ultrasonic imaging, ultrasonic contact is required. Such contact may be either through a water medium or by substantially direct contact with body tissue.

An ultrasonic step scanning device typical of those used for ultrasonic probing is described in Plesset, U.S. Patent 4,409,982, dated October 18, 1983. This patent describes an electronically step-scanned real-time ultrasonic imaging system which includes a transducer assembly having an array of elements, each with an axis of transmission along which ultrasonic energy is transmitted when electrically stimulated. The disclosure of U.S. Patent 4,409,982 is incorporated herein by reference.

Another ultrasonic prostate probe assembly of the type known to the prior art is disclosed in Dow, et al., U.S. Patent 4,841,979, dated June 27, 1989. This patent also discloses an extensive list of prior art purporting to describe examples of ultrasonic transducer probe assemblies. The disclosure of U.S. Patent 4,841,979 is also incorporated herein by reference.

As indicated in U.S. Patent 4,841,979, space limitations and restrictions on ability to manipulate the probe pose significant problems to satisfactory probing. The Dow, et al., ultrasonic probe assembly attempts to respond to the problems of probe use in situations involving space constraints by providing an ultrasonic prostate probe assembly, wherein the ultrasonic transducer is carried on a Gimbal cup rotatably mounted to the probe assembly to allow selection of multiple scan directions when the probe is used. In such an arrangement, the transducer is pivoted by a spring biased flexible cable linkage connected to a linear motor with an electric coil as a reciprocating moving member. Such a device is obviously complicated and expensive and may have reliability problems because of the complexity.

A disadvantage of the Dow, et al., ultrasonic prostate probe assembly and of other probes known to the prior art is the difficulty of accommodating the probe to the pelvic anatomy of the patient. In addition, in a device such as described by Dow, et al., it is difficult to adapt a biopsy probe to the device because of the need to maintain the forward end of the probe assembly free to rotate and move in the directions necessary to achieve proper scanning of the body tissue. A biopsy probe, such as is disclosed in Cooper, U.S. Patent 4,838,506, dated June 13, 1989, is often very useful in facilitating a complete examination of the prostate. The disclosure of U.S. Patent 4,838,506 is also incorporated herein by reference.

It is apparent, therefore, that a substantial need exists for an ultrasonic probe apparatus which is able to be easily positioned within the pelvic anatomy of the patient and which will provide an ultrasonic field of view encompassing the patient's substantially entire prostate.

## Summary of the Invention

The present invention relates to an ultrasonic probe apparatus and method of using same for probing a patient's interior cavity to examine, for example, a prostate or vaginal cavity. The invention provides a probe which is adapted to conform to the patient's pelvic anatomy but which additionally is capable of imaging the entire area to be examined, such as the patient's entire prostate. The invention is also able to associate a biopsy probe with the apparatus for use as needed.

According to the present invention, there is provided an ultrasonic probe apparatus capable of imaging a patient's entire prostate or other area by substantially direct contact with body tissue, which includes a housing having a straight portion and a curvilinear end portion with a convexly shaped outer surface, preferably configured to describe an arc of a circle. The end portion is thus shaped to conform to a patient's pelvic anatomy and is adapted to be inserted thereinto, so that the outer surface of the curvilinear end portion may be positioned to face the prostate. Ultrasonic transducer means which generate and receive ultrasonic waves in a plurality of diverging transmission axes are arrayed along the outer surface of the curvilinear end portion to a sufficient extent to enable the diverging ultrasonic transmissions to provide a field of view of the patient's substantially entire prostate, thus enabling a comprehensive image of the prostate to be obtained. In one embodiment of the invention, the apparatus further comprises a biopsy probe means for obtaining a biopsy sample of prostate tissue and means to secure the biopsy probe to the straight portion of the housing.

In one aspect of the invention there is provided a method of ultrasonically probing a patient's prostate comprising the steps of: inserting into the rectal cavity, of the patient, a convexly curved linear ultrasonic array having a sectoral field of view; positioning the array in substantially direct contact with the rectal wall so that the substantially entire prostate of the patient lies within the field of view; and operating the array to acquire imaging data.

Also provided in accordance with the invention is a method of ultrasonically probing a patient's prostate comprising providing an ultrasonic probe apparatus of the type previously described, having a housing which includes a straight portion and a curvilinear end portion with an outer surface configured to describe an arc of a circle, the end portion being shaped to conform to a patient's pelvic anatomy and being adapted to be inserted thereinto, and with ultrasonic transducer means for generating and receiving ultrasonic waves in a plurality of diverging transmission axes arrayed along the outer surface of the curvilinear end portion to a sufficient extent to enable the diverging ultrasonic transmissions to encompass the patient's substantially entire prostate; introducing the probe into the patient's pelvic anatomy, orienting the probe so that the outer surface of the curvilinear end portion faces the prostate and directing the ultrasonic transmissions to the prostate to provide a field of view of substantially the entire prostate and producing an image of the prostate in response to the generated and received ultrasonic waves.

## Description of the drawings

The above-described and other features of the invention will become apparent upon reference to the drawings, wherein:

FIG. 1 is a schematic view of an ultrasonic probe typical of the prior art;

FIG. 2 is a schematic view of the ultrasonic probe apparatus in accordance with the invention, as shown in use; and

FIG. 3 is a schematic view of the presently preferred design of the ultrasonic probe apparatus in accordance with the invention, also showing a schematic view of a biopsy probe which may be used therewith.

## Detailed Description of the Preferred Embodiment

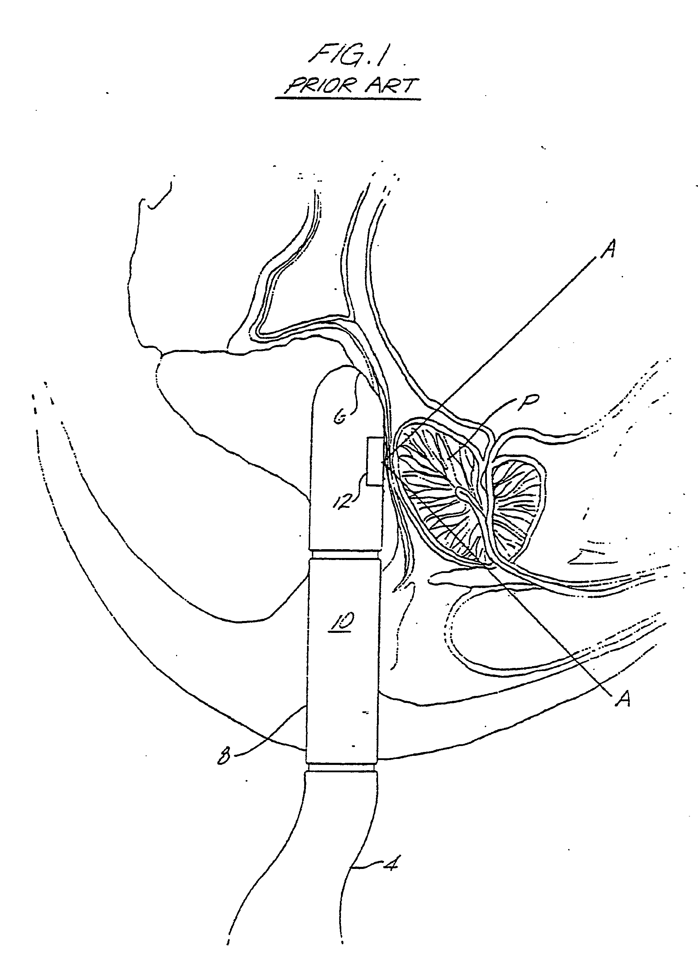

Referring to the drawings, FIG. 1 shows a typical ultrasonic probe of the type which has been used prior to the present invention. As seen in FIG. 1, the probe 10 comprises an elongated housing 8 which terminates in a rounded end 6, to facilitate penetration into the pelvic anatomy. At the opposite end, the probe 10 is connected to a flexible connecting means 4. Ultrasonic transducer means 12 are located along a lateral surface of the housing that is to be inserted into the patient. A typical field of view shown by lines A-A is intended to encompass as much of the prostate (designated P) as is possible. However, the field of view may not include all of the prostate, especially if the prostate is enlarged. Also, it is apparent that there is no convenient path for the passage of a biopsy needle or for the inclusion of a biopsy probe along side the housing 8 of the probe 10, which would permit the biopsy needle to be directed to the prostate.

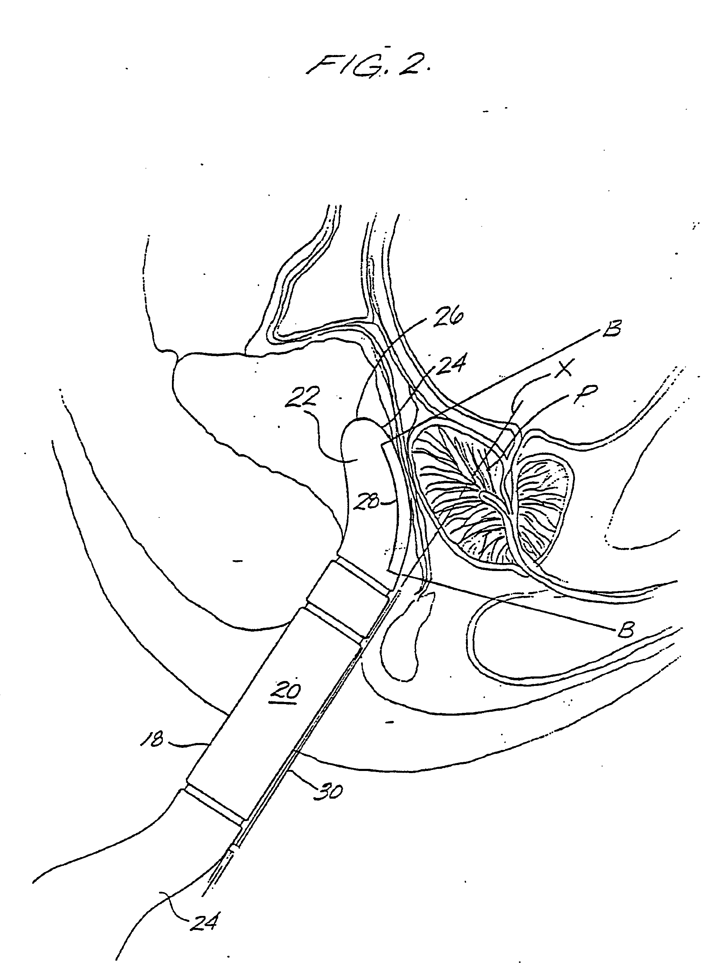

In contrast to the prior art ultrasonic probe shown in FIG. 1, the ultrasonic probe 20 in accordance with the apparatus of the invention, as shown in FIG. 2, comprises a housing having a straight portion 18 and a curvilinear end portion 22 having a convexly shaped outer surface 24. Outer surface 24 is preferably configured to describe an arc of a circle. A rounded end 26 facilitates insertion into the patient inasmuch as it conforms to the patient's pelvic anatomy.

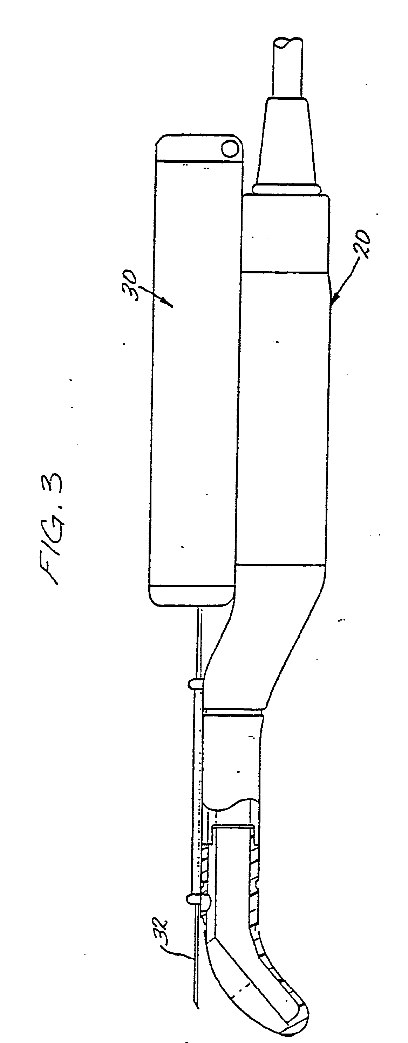

Provided along the outer surface 24 of the curvilinear end portion, which may be oriented to face the prostate, is ultrasonic transducer means 28 for generating and receiving ultrasonic waves. As shown in FIG. 2, the ultrasonic transducer means is arrayed along the outer surface 24 of the curvilinear end portion 22 in direct contact with the rectal wall behind which the prostate lies. At the opposite end of the probe apparatus 20 is flexible connecting means 24. In the preferred embodiment illustrated in FIG. 3, a biopsy probe 30 is provided which is secured to the housing of the ultrasonic probe apparatus along the straight portion 18 of the housing thereof and, as illustrated, may be directed toward the prostate when the probe is positioned within the patient's pelvic anatomy. The biopsy needle 32 of the probe will pass through the rectal wall and enter the prostate as shown by the dotted line "X" extending from biopsy probe 30.

In accordance with the invention, ultrasonic transmission means are provided which generate and receive ultrasonic waves in a plurality of diverging transmission axes. In this way, the field of view shown by lines B-B of the ultrasonic probe apparatus includes all of the prostate and surrounding tissue. The field of view is also broad enough to include an enlarged prostate.

Preferably, the ultrasonic transducer means comprise a curved linear array that permits scanning of areas beneath or between bone or other obstructions with the field of view sufficiently large for the intended purpose. One suitable device is Model 2100-0068-1A, available from Acoustic Imaging, Tempe, Arizona. It has been found that a curved array of 7.5 mH$_z$ with a 40-mm radius of curvature providing a 33-mm chord and a 60° angle of view is generally sufficient to provide a field of view to encompass the entire prostate, so that the prostate can be imaged when the probe contacts the rectal wall. The aforementioned U.S. application S.N. 07/443,752, although directed to a rotatable linear array, describes an overall array imaging system of the type which may be used in accordance with the present invention.

A comparison of the prior art and the invention is seen by comparing FIGS. 1 and 2. The prior art device shown in FIG. 1 uses a mechanical section transducer or phased array transducer generating a 90° field of view going down to a central point mounted on a straight housing. The prior art device is not configured so as to be anatomically compatible with the pelvic anatomy of the patient. Furthermore, with such a device, in order to provide an image of the complete prostate, it is necessary to use a water delay stand-off for examination. The present invention, in contrast, provides a simple and expedient ultrasonic probe apparatus that may be used for examination of the patient without the need to employ a water delay stand-off because the transducer means directly contacts tissue surface, e.g., the rectal wall.

It is apparent from the foregoing that various changes and modifications may be made without departing from the invention. For example, as previously mentioned, the ultrasonic probe may be used as a type of vaginal probe because of its suitable geometry for such applications as ovum harvesting which is a procedure used by those unable to conceive normally. Accordingly, the scope of the invention should be limited only by the appended claims, wherein:

## Claims

1. An ultrasonic probe apparatus capable of imaging a patient's entire prostate comprising:

   a housing having a straight portion and a curvilinear end portion with an outer surface configured to describe an arc of a circle,

   said end portion being shaped to conform to a patient's pelvic anatomy and being adapted to be inserted thereinto so that the outer surface of said curvilinear end portion may be positioned to face the prostate,

   ultrasonic transducer means for generating arid receiving ultrasonic waves in a plurality of diverging transmission axes arrayed along said outer surface of said curvilinear end portion to a sufficient extent to enable said diverging ultrasonic transmissions to provide a field of view of the patient's substantially entire-prostate, thereby enabling a comprehensive image of the prostate to be obtained.

2. An ultrasonic probe apparatus according to claim 1 wherein said ultrasonic transducer means comprises curved array of 7.5 mH$_z$ center frequency with a 40-mm radius of curvature providing a 33-mm chord and a 60° angle of view.

3. An ultrasonic probe apparatus according to claim 1 further comprising biopsy probe means for obtaining a biopsy sample of prostate tissue and means to secure the biopsy probe to the straight portion of the housing.

4. An ultrasonic probe apparatus according to claim 3 wherein said biopsy probe includes a biopsy needle and said biopsy probe is so aligned with respect to the housing of the ultrasonic probe apparatus that the needle may be inserted into the prostate while the ultrasonic probe apparatus is in place in the patient's pelvic anatomy to obtain a prostate tissue sample.

5. An ultrasonic probe apparatus comprising:

   a housing having a straight portion and a curvilinear end portion with an outer surface configured to describe an arc of a circle;

   said end portion being shaped to conform to a patient's pelvic anatomy and being adapted to be inserted thereinto so that the outer surface of said curvilinear end portion may be positioned to face the area to be examined;

   ultrasonic transducer means for generating and receiving ultrasonic waves in a plurality of diverging transmission axes arrayed along said outer surface of said curvilinear end portion to a sufficient extent to enable said diverging ultrasonic transmissions to provide a field of view of the entire area to be examined, thereby enabling a comprehensive image of the area to be obtained.

6. An ultrasonic probe apparatus according to claim 5 wherein said ultrasonic transducer means comprises curved array of 7.5 mH$_z$ center frequency with a 40-mm radius of curvature providing a 33-mm chord and a 60° angle of view.

7. An ultrasonic probe apparatus according to claim 5 further comprising biopsy probe means for obtaining a biopsy sample of tissue and means to secure the biopsy probe to the straight portion of the housing.

FIG.1
PRIOR ART

*FIG. 2.*

FIG. 3

EP 0 446 645 A1

EP 0 446 645 A1

European Patent Office

**EUROPEAN SEARCH REPORT**

Application Number

EP 91 10 2166

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | US-A-3 942 530 (A. NORTHEVED)<br>* column 2, lines 17-62; figures 3,4 * | 1 | A 61 B 8/12 |
| A | | 3-5,7 | |
| Y | EP-A-0 123 594 (UNIVERSITE FRANCOIS RABELAIS)<br>* page 4, line 5 - page 6, line 30; figures 1-3 * | 1 | |
| A | | 2,5,6 | |
| Y | EP-A-0 061 332 (OLYMPUS OPTICAL CO., LTD.)<br>* page 6, line 15 - page 8, line 4; figures 1,2 * | 1 | |
| A | | 5 | |
| A | EP-A-0 273 180 (SIEMENS AG)<br>* column 1, lines 1-5; column 11, line 37 - column 13, line 24; figure 5 * | 1,3 | |

TECHNICAL FIELDS SEARCHED (Int. Cl.5)

A 61 B 8/00
A 61 B 10/00
G 01 S 7/00

The present search report has been drawn up for all claims

| Place of search | Date of completion of the search | Examiner |
|---|---|---|
| BERLIN | 06-05-1991 | WEIHS J.A. |